# EUROPEAN PATENT SPECIFICATION

(11) **EP 3 890 647 B1**
(45) Date of publication and mention of the grant of the patent: **08.03.2023**
(21) Application number: 19831795.0
(22) Date of filing: 03.12.2019
(51) Int. Cl.: A61B 90/80, A61F 13/02, A61M 37/00, A61N 1/04, A61N 1/30, A61B 17/00

(54) **SKIN PREPARATION PATCHES**
HAUTPRÄPARATIONSPFLASTER
PATCH DE PRÉPARATION DE LA PEAU

(30) Priority: 03.12.2018 GB 201819737
(43) Date of publication of application: 13.10.2021
(73) Proprietor: Dermal Diagnostics Limited, Loughborough, Leicestershire LE11 3QF (GB)
(72) Inventor: CHOWDHURY, Dewan Fazlul Hoque, Leicestershire LE11 1PN (GB)
(74) Representative: Serjeants LLP
(86) International application number: PCT/GB2019/053408
(87) International publication number: WO 2020/115465

(56) References cited:
- US-A1- 2005 106 226
- US-A1- 2007 088 248
- US-A1- 2008 274 166
- US-A1- 2014 228 736

## Description

### Technical field

The invention relates to the field of transdermal devices for delivering drugs or obtaining samples through the skin of a human or animal subject. In particular, the invention relates to a patch that is applied first to the skin in order to facilitate preparation of the skin for the transdermal procedure.

### Background of the invention

It is well known to use transdermal procedures for obtaining fluid samples or other analytes from a subject by withdrawal through the skin without the use of hypodermic needles. One example is the monitoring of glucose levels by persons who are diabetic. It is similarly known to use transdermal procedures to deliver drugs or other biologically active substances into the body of a subject. Electrochemical techniques such as iontophoresis or reverse iontophoresis may be used to enhance the transport of the substances or analytes in question across the skin.

Before the device that will carry out the transdermal procedure is applied to the skin, it is typically necessary to prepare the skin in some way to increase the effectiveness, safety or reliability of the procedure. Most simply, the skin may need to be cleaned or sterilized to prevent infection when transdermal channels are opened. Other preparation techniques can be used to enhance the permeability of the skin, for example by abrading the skin or applying a chemical permeant. A variety of poration techniques can also be used to open channels through the stratum corneum, for example by electroporation or applying microneedles to the surface of the skin. If electrochemical techniques are to be used, then it may be necessary to apply a liquid or gel electrolyte to the skin to improve conductivity between the skin and the electrodes of the device.

The working area of a transdermal device, within which the transdermal process occurs, is typically quite small relative to the total area of the device that is placed in contact with the skin. It is therefore important to ensure that the device is correctly located so that the working area of the device is aligned with the area of the skin in which the preparation process has been carried out. Misalignment between the prepared skin area and the electrodes or other components in the working area of the device can result in poor transdermal transport and/or unreliable measurement of analyte levels by the device. The working area of a transdermal device is typically somewhere in the centre of the face that is applied to the skin so that, while it is being applied, the body of the device hides both the working area and the skin preparation area from view, making difficult the accurate location of the device on the skin.

Published patent application US 2005/0106226 A1 discloses a drug delivery system comprising a pre-treatment patch adapted to be placed on a patient's skin, which includes a skin template that remains on the skin after the pre-treatment patch is removed. A gel patch containing a hydrogel formulation may then be applied to the pre-treated skin area.

Published patent application US 2014/0228736 A1 discloses a patch system for providing an alignment mechanism which facilitates the application of an integrated poration device and then the subsequent step of applying a drug reservoir patch over the area in which the micropores are formed. The two components may be linked on the same substrate, wherein a folding process can be utilized to bring the drug reservoir into contact with the porated skin area. The top and bottom of the porator array may be protected by release liners. An adhesive causes an outer portion of the porator array to remain in place when the poration elements are pulled away from the skin. The drug reservoir patch is constructed of a size to fit within the area left behind following removal of the poration elements.

Published patent application US 2008/0274166 A1 discloses a patch for transdermal drug delivery, which comprises an elongated support structure configured shaped to define a drug delivery area and a window area separated by a flexible fold line. The window is surrounded by a frame with adhesive on both sides thereof. A first liner protects the lower side of the window frame and is removed to reveal the window area of the support structure before adhering the patch to the skin. A second liner covers the drug delivery area and protects the upper side of the window frame, while leaving a portion of the skin exposed through the window area of the support structure. The subject applies an applicator to a portion of skin exposed through the window to increase its permeability. The subject then pulls a tab on the second liner to separate it from the window frame, uncover the drug delivery area and cause the support structure to flex along the fold line and bring the drug delivery into contact with the portion of skin exposed through the window area.

Published patent application US 2007/0088248 A1 discloses devices designed to disrupt a defined area of skin, which approximates the area that a patch for a therapeutic agent is designed to contact. Some of the devices employ a mask, which is integrated with the disrupting member and defines the area to be disrupted. The device may be adhered to the skin following removal of an adhesive backing.

### Summary of the invention

The invention provides a kit comprising a skin preparation patch and a transdermal device, as defined in claim 1.

The invention further provides a skin preparation patch as defined in claim 6.

Preferred but non-essential features of the invention are defined in the dependent claims.

The skin preparation patch according to the invention serves a dual purpose. The second part defines the area of the skin in which the required preparation process should be carried out. The second part can then be removed to leave the first part adhered to the skin, which can be used as a guide for correctly locating the transdermal device with the working area of the device and the prepared area of the skin in alignment.

The skin treatment device may itself be in the form of a patch and its periphery may need to be hermetically sealed against the skin so as to prevent the loss of moisture from the working area, which could lead to a detrimental impact on the ability of an analyte to diffuse through or react in the working area. The patch according to the invention can ensure that sufficient area is provided around the working area of the device to accommodate such a seal.

In this specification, the term "underside" and cognate words are used to refer to the side of a patch or device that, in use, is closest to the skin of a subject. It will be understood that the patch and device may be used in any orientation, depending on the body part to which they are applied, and may similarly be manufactured, transported or stored in any orientation, without departing from the scope of the invention defined by the claims.

### The drawings

Figure 1 illustrates a skin preparation patch according to a first embodiment of the invention when it is first applied to the skin.
Figure 2 illustrates the patch of Figure 1 following removal of a third part.
Figure 3 illustrates the patch of Figure 2 following preparation of the skin.
Figure 4 illustrates the patch of Figure 3 following and removal of a second part.
Figure 5 illustrates the use of a first part of the patch of Figure 1 to locate a transdermal device.
Figure 6 illustrates a skin preparation patch according to a second embodiment of the invention.
Figure 7 illustrates the use of a first part of the patch of Figure 6 to locate a transdermal device.
Figure 8 illustrates a two-part skin preparation patch, which does not fall within the scope of the claimed invention.

The skin preparation patch shown in claim 1 is formed from a thin, flexible material such as a polymer foam, or other polymeric membrane, produced from a range of materials such as silicone, acrylic polymers, cellulose acetate, etc. It has an adhesive layer over the entire area of its underside. Suitable adhesives for temporarily attaching patches to skin, so that they can be peeled off after use, are well known. The adhesive layer is protected by a backing sheet/release liner (not shown) that is peeled off immediately before use of the patch.

The patch is divided by cut lines into three parts 1,2,3. The adhesive layer will generally be sufficient to hold the respective parts together by their edges after removal of the backing sheet but small connections between the parts could be left for this purpose, provided that they can easily be torn when separation of the parts is desired.

A first part 1 follows the perimeter of the patch and surrounds the second and third parts 2,3. The first part 1 may form a continuous loop or may be cut by one or more lines or tabs 4 to make easier the eventual removal of any of the parts from the skin. A second part 2 comprises the whole interior of the patch, except for a central area that is formed by the third part 3.

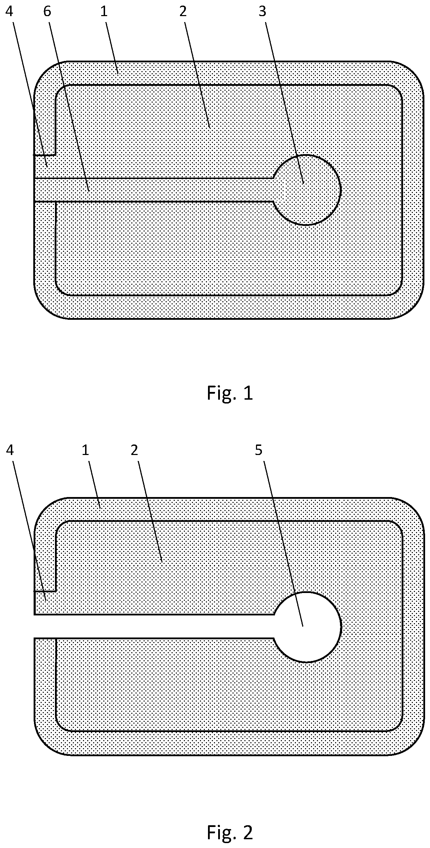

Figure 2 shows the patch following removal of the "keyhole" shaped third part 3 to leave an opening through the patch to the skin. The opening comprises a circular area 5, bounded by the second part 2, which defines the area of the skin that needs to be prepared for the transdermal procedure. A strip 6 of the third part 3 extends to the perimeter of the second part 2, whereby it is easy for the user to grip the end of the strip 6 and to remove the third part 3 from the patch by pulling on the strip 6. This ensures that the fingers of the user are kept away from skin preparation area, which needs to be kept clean and may have been cleansed prior to application of the patch.

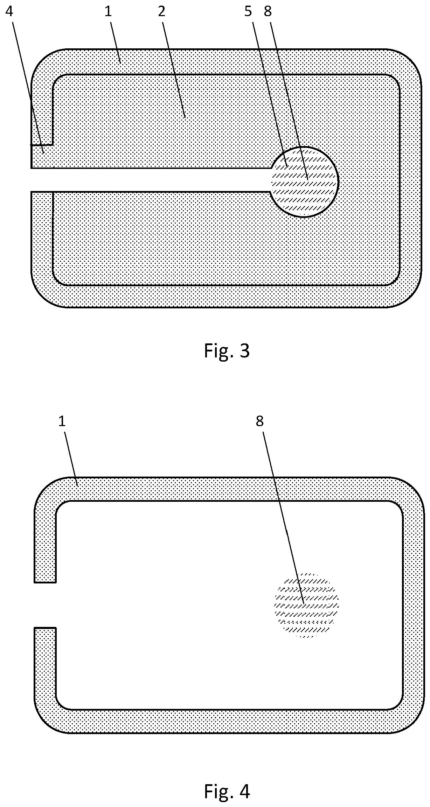

Figure 3 is similar to Figure 2 but shows the area of skin 8 that has been prepared for the transdermal procedure by accessing it through the circular part of the opening 5. The preparation may involve applying a tool (not shown) that has microneedles or other micro-protrusions to abrade the skin or form pores through the stratum corneum. Additionally or alternatively, it may involve applying a chemical substance such as a permeant or a cleaning or sterilizing agent to the skin preparation area. If the transdermal device uses electrodes, then a final step may involve applying an electrolyte to the skin treatment area in liquid or gel form to enhance the conductivity between the skin and the electrodes. During the skin preparation procedure, the second part 2 of the patch masks areas of the skin other than the intended skin preparation area.

Following the preparation step, the user removes the second part 2 of the patch by unpeeling it from anywhere on its perimeter. (One of the free edges where the strip 6 has been removed is likely to be easiest.) That leaves just the first part 1 of the patch adhered to the skin, forming a frame, with the treated area of skin 8 at a defined location inside it, as shown in Figure 4.

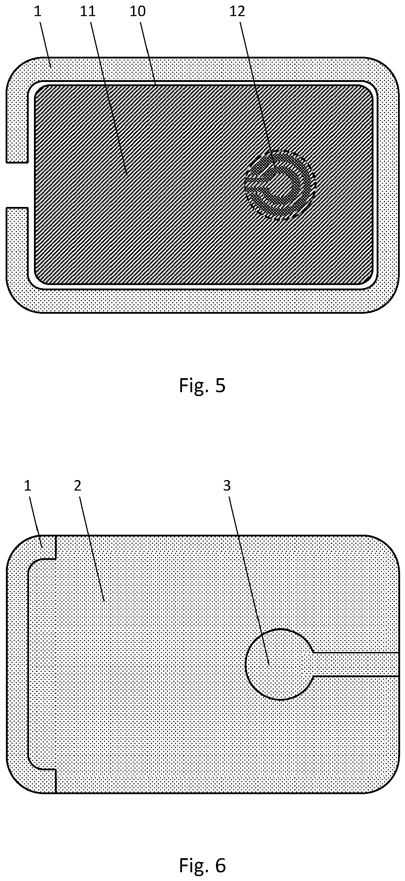

Figure 5 illustrates how the frame formed by the first part 1 of the patch can be used to locate a transdermal device 10 correctly on the skin. The device 10 has a working area 12 on its underside, shown schematically here by a set of electrodes, which lies within and is smaller than the overall skin contact area 11 of the device. The working area 12 becomes automatically aligned with the prepared area of skin 8 when the transdermal device 10 is located within the frame. The device 10 may be held in contact with the skin in any conventional manner, for example by an adhesive layer on its underside or by means of a strap (not shown). The first part 1 of the patch can be removed from the skin at any time after the device 10 has been located.

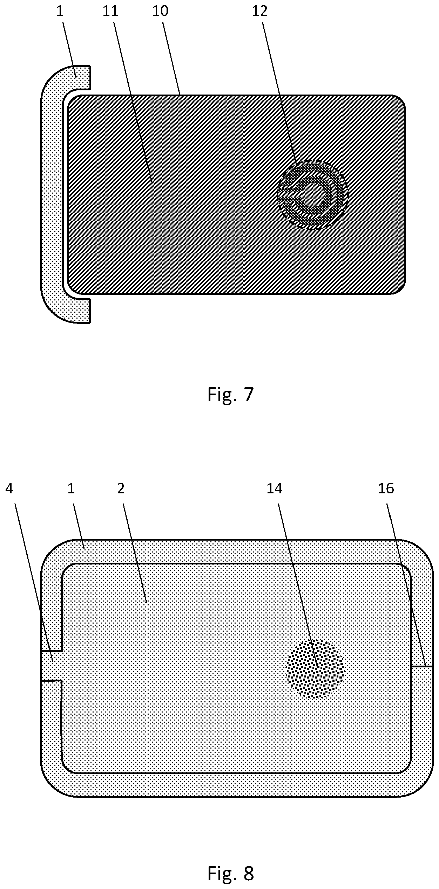

Figure 6 illustrates a second embodiment of the invention, which is similar to the first embodiment, except that the first part 1 of the patch does not surround the second part 2. Instead, it extends around only two comers of the generally rectangular perimeter of the patch. Figure 7 illustrates how the first part 1 of the patch of Figure 6 is nevertheless sufficient to locate the device 10 in its correct alignment. In fact, a first part 1 of the patch having only a single comer would still be sufficient for this purpose. It will be understood that other arrangements are also possible, for example a first part with no comers but with printed marks on its upper surface to indicate the correct location of the device 10.

Figure 8 shows a further example of a skin preparation patch, which does not fall within the scope of the claimed invention.. In this example, the patch has no third part that can be removed to form a skin preparation opening. Instead, the skin preparation (skin-prep) area is defined by skin preparation means 14 on a defined area of the underside of the second part 2. Preferably, the skin preparation means 14 consists of an array of microneedles or other micro-protrusions that can be pressed against the surface of the skin, for example by pushing a roller tool (not shown) across the upper surface of the patch. However, any of the aforementioned physical or chemical preparation means may similarly be provided in a defined area of the patch.

In the case of physical means such as micro-protrusions, the skin preparation area may be identified as subsections, such as two semi-circles for example, either with lettering or numbering to identify where the skin preparation means is to be applied. This is significant in the case of the use of a micro-protrusion patch for example, where it is known that application of force directly in the centre of a skin-prep patch greater than 5mm in diameter leads to minimal effect on the periphery as compared to the centre or the region where the most pressure is applied. Therefore demarcating the area into two or more areas can allow the skin-prep to be applied in more than one step, in the case where the skin-prep patch is applied to the underside of the aforementioned device. This will allow a user to apply force with a finger or thumb, or an implement provided, over smaller regions, thus providing a higher and more concentrated force over smaller areas, significantly enhancing the effectiveness of the skin-prep step. The second part 2 of the patch may thereafter be removed to reveal a situation identical to Figure 4.

The reader will understand that the shape of the patch is not important in itself but it should be matched to the shape and configuration of the transdermal device with which the patch is to be used. In particular, the internal contour of the first part of the patch should match some or all of the outline of the device, while the second part of the patch should define an opening or an area of skin preparation means that substantially matches the shape, size and location of the working area of the device.

It is not essential that the third part 3 of the patch should be provided with a strip 6 extending to the perimeter of the second part 2 but it is preferred that some sort of tab should be provided to make the part 3 easy to grip. Similar tabs may be provided on the other parts 1,2 if desired. It is strongly preferred but not essential that the third part 3 should be completely detachable from the second part. One can imagine that if the third part 3 were peeled back from the circular end, the long strip 6 would allow the third part 3 to be held well clear of the skin preparation area 8 without removing it completely. Any of the first, second or third parts may be subdivided for ease of use or manufacture. An example of a cut line 16 subdividing the first part 1 is shown in Figure 8.

It is not essential for the adhesive layer to cover the entire area of the underside of the patch. As the final part of the patch to be removed, the first part 1 does need to be provided with an adhesive layer. However, if the adhesion of the first part 1 is sufficiently strong, and if there is sufficient mutual attachment between the various parts 1,2,3, e.g. by the provision of small connecting links, then it might be possible to omit the adhesive layer from the second and/or third parts 2,3.

## Claims

1. A skin preparation kit comprising:
a transdermal device (10) having a skin contact area (11) and a working area (12) that lies within and is smaller than the skin contact area (11); and
a skin preparation patch comprising a first part (1), a second part (2) and a third part (3), wherein:
at least the first part (1) of the patch comprises an adhesive layer suitable for adhering the patch to skin (8);
the third part (3) can be removed, thereby to create an opening in the second part (2);
the opening in the second part (2) of the patch defines a skin preparation area (5) that lies within and is smaller than the overall area of the second part (2); and
the second part (2) can be removed to leave the first part (1) adhered to the skin (8);
whereby the first part (1) of the patch defines a desired location for placing the transdermal device in contact with the skin (8) such that the skin preparation area (5) defined by the second part (2) of the patch prior to its removal is in alignment with the working area (12) of the device (10);
**characterized in that**:
the skin preparation patch is divided by cut lines into the first part (1), the second part (2) and the third part (3); and
the third part (3) can be removed while leaving the first and second parts (1,2) of the patch adhered to the skin (8).

2. A skin preparation kit according to claim 1, wherein the third part (3) comprises an inner portion in the shape of the skin preparation area (5), which lies wholly inside the perimeter of the second part (2), and a strip (6) that connects the inner portion to the perimeter of the second part (2).

3. A skin preparation kit according to claim 1 or claim 2, wherein the second part (2) of the patch further comprises an adhesive layer.

4. A skin preparation kit according to any preceding claim, wherein the first part (1) of the patch surrounds the second part (2).

5. A skin preparation kit according to any preceding claim, wherein the first part (1) of the patch comprises at least one internal comer for defining the desired location of the transdermal device (10) in contact with the skin (8).

6. A skin preparation patch comprising a first part (1), a second part (2) and a third part (3), wherein:
at least the first part (1) comprises an adhesive layer suitable for adhering the patch to skin (8);
the third part (3) can be removed, thereby to create an opening in the second part (2) that defines a skin preparation area (5); and
the second part (2) can be removed to leave the first part (1) adhered to the skin (8);
**characterized in that** the skin preparation patch is divided by cut lines into the first part (1), the second part (2) and the third part (3).

7. A skin preparation patch according to claim 6, wherein the third part (3) comprises:
an inner portion in the shape of the skin preparation area (5), which lies wholly inside the perimeter of the second part (2); and
a strip (6) that connects the inner portion to the perimeter of the second part (2).

8. A skin preparation patch according to claim 6 or claim 7, wherein at least the first and second parts (1,2) of the patch comprise the adhesive layer.

9. A skin preparation patch according to any of claims 6 to 8, wherein the first part (1) of the patch surrounds the second part (2).

10. A skin preparation patch according to any of claims 6 to 9, wherein the first part (1) of the patch comprises at least one internal comer for defining the desired location of a transdermal device (10) to be placed in contact with the skin (8).

## Patentansprüche

1. Hautpräparationskit; umfassend:
eine transdermale Vorrichtung (10), die eine Hautkontaktfläche (11) und eine Arbeitsfläche (12), die darin liegt und kleiner ist als die Hautkontaktfläche (11), aufweist; und
Hautpräparationspflaster, das einen ersten Teil (1), einen zweiten Teil (2) und einen dritten Teil (3) umfasst, wobei:
mindestens der erste Teil (1) des Pflasters eine Klebeschicht umfasst, die geeignet ist zum Ankleben des Pflasters auf Haut (8);
der dritte Teil (3) entfernt werden kann, um dadurch eine Öffnung in dem zweiten Teil (2) zu erstellen;
die Öffnung in dem zweiten Teil (2) des Pflasters eine Hautpräparationsfläche (5) definiert, die darin liegt und kleiner ist als die Gesamtfläche des zweiten Teils (2); und
der zweite Teil (2) entfernt werden kann, um den ersten Teil (1) auf der Haut (8) angeklebt zu lassen;
wobei der erste Teil (1) des Pflasters eine gewünschte Stelle zum Platzieren der transdermalen Vorrichtung in Kontakt mit der Haut (8) so definiert, dass die durch den zweiten Teil (2) des Pflasters definierte Hautpräparationsfläche (5) vor ihrer Entfernung an der Arbeitsfläche (12) der Vorrichtung (10) ausgerichtet ist;
**dadurch gekennzeichnet, dass**:
das Hautpräparationspflaster durch Schnittlinien in den ersten Teil (1), den zweiten Teil (2) und den dritten Teil (3) unterteilt ist; und
der dritte Teil (3) entfernt werden kann, während der erste und der zweite Teil (1,2) des Pflasters an der Haut (8) angeklebt bleiben.

2. Hautpräparationskit nach Anspruch 1, wobei der dritte Teil (3) einen inneren Abschnitt in der Form der Hautpräparationsfläche (5), der vollständig innerhalb des Umfangs des zweiten Teils (2) liegt, und einen Streifen (6), der den inneren Abschnitt mit dem Umfang des zweiten Teils (2) verbindet, umfasst.

3. Hautpräparationskit nach Anspruch 1 oder Anspruch 2, wobei der zweite Teil (2) des Pflasters weiter eine Klebeschicht umfasst.

4. Hautpräparationskit nach einem vorstehenden Anspruch, wobei der erste Teil (1) des Pflasters den zweiten Teil (2) umgibt.

5. Hautpräparationskit nach einem vorstehenden Anspruch, wobei der erste Teil (1) des Pflasters mindestens eine Innenecke zum Definieren der gewünschten Stelle der transdermalen Vorrichtung (10) in Kontakt mit der Haut (8) umfasst.

6. Hautpräparationspflaster, das einen ersten teil (1), einen zweiten teil (2) und einen dritten teil (3) umfasst, wobei:
mindestens der erste Teil (1) eine Klebeschicht umfasst, die geeignet ist zum Ankleben des Pflasters auf Haut (8);
der dritte Teil (3) entfernt werden kann, um dadurch eine Öffnung in dem zweiten Teil (2) zu erstellen, die eine Hautpräparationsfläche (5) definiert; und
der zweite Teil (2) entfernt werden kann, um den ersten Teil (1) auf der Haut (8) angeklebt zu lassen;
**dadurch gekennzeichnet, dass** das Hautpräparationspflaster durch Schnittlinien in den ersten Teil (1), den zweiten Teil (2) und den dritten Teil (3) unterteilt ist.

7. Hautpräparationspflaster nach Anspruch 6, wobei der dritte Teil (3) Folgendes umfasst:
einen inneren Abschnitt in der Form der Hautpräparationsfläche (5), der vollständig innerhalb des Umfangs des zweiten Teils (2) liegt; und
einen Streifen (6), der den inneren Abschnitt mit dem Umfang des zweiten Teils (2) verbindet.

8. Hautpräparationspflaster nach Anspruch 6 oder Anspruch 7, wobei mindestens der erste und der zweite Teil (1,2) des Pflasters die Klebeschicht umfassen.

9. Hautpräparationspflaster nach einem der Ansprüche 6 bis 8, wobei der erste Teil (1) des Pflasters den zweiten Teil (2) umgibt.

10. Hautpräparationspflaster nach einem der Ansprüche 6 bis 9, wobei der erste Teil (1) des Pflasters mindestens eine Innenecke zum Definieren der gewünschten Stelle einer transdermalen Vorrichtung (10), die in Kontakt mit der Haut (8) zu platzieren ist, umfasst.

## Revendications

1. Kit de préparation de la peau comprenant :
un dispositif transdermique (10) présentant une zone de contact avec la peau (11) et une zone de travail (12) qui se trouve dans la zone de contact avec la peau (11) et qui est plus petite que celle-ci ; et
timbre de préparation de la peau comprenant une première partie (1), une deuxième partie (2) et une troisième partie (3), dans lequel :
au moins la première partie (1) du timbre comprend une couche adhésive appropriée pour coller le timbre à la peau (8) ;
la troisième partie (3) peut être retirée, ce qui permet de créer une ouverture dans la deuxième partie (2) ;
l'ouverture dans la deuxième partie (2) du timbre définit une zone de préparation de la peau (5) qui se trouve dans la zone globale, et qui est plus petite que celle-ci, de la deuxième partie (2) ; et
la deuxième partie (2) peut être retirée pour laisser la première partie (1) collée à la peau (8) ;
selon lequel la première partie (1) du timbre définit un emplacement souhaité pour placer le dispositif transdermique en contact avec la peau (8) de telle sorte que la zone de préparation de la peau (5) définie par la deuxième partie (2) du timbre avant son retrait soit en alignement avec la zone de travail (12) du dispositif (10) ;
**caractérisé en ce que** :
le timbre de préparation de la peau est divisé par des lignes de coupe dans la première partie (1), la deuxième partie (2) et la troisième partie (3) ; et
la troisième partie (3) peut être retirée, tout en laissant les première et deuxième parties (1, 2) du timbre collées à la peau (8).

2. Kit de préparation de la peau selon la revendication 1, dans lequel la troisième partie (3) comprend une partie interne sous la forme de la zone de préparation de la peau (5), qui se trouve entièrement à l'intérieur du périmètre de la deuxième partie (2), et une bande (6) qui relie la partie interne au périmètre de la deuxième partie (2).

3. Kit de préparation de la peau selon la revendication 1 ou la revendication 2, dans lequel la deuxième partie (2) du timbre comprend en outre une couche adhésive.

4. Kit de préparation de la peau selon une quelconque revendication précédente, dans lequel la première partie (1) du timbre entoure la deuxième partie (2).

5. Kit de préparation de la peau selon une quelconque revendication précédente, dans lequel la première partie (1) du timbre comprend au moins un coin interne pour définir l'emplacement souhaité du dispositif transdermique (10) en contact avec la peau (8).

6. Timbre de préparation de la peau comprenant une première partie (1), une deuxième partie (2) et une troisième partie (3), dans lequel :
au moins la première partie (1) comprend une couche adhésive appropriée pour coller le timbre à la peau (8) ;
la troisième partie (3) peut être retirée, ce qui permet de créer une ouverture dans la deuxième partie (2) qui définit une zone de préparation de la peau (5) ; et
la deuxième partie (2) peut être retirée pour laisser la première partie (1) collée à la peau (8) ;
**caractérisé en ce que** le timbre de préparation de la peau est divisé par des lignes de coupe dans la première partie (1), la deuxième partie (2) et la troisième partie (3).

7. Timbre de préparation de la peau selon la revendication 6, dans lequel la troisième partie (3) comprend :
une partie interne sous la forme de la zone de préparation de la peau (5), qui se trouve entièrement à l'intérieur du périmètre de la deuxième partie (2) ; et
une bande (6) qui relie la partie interne au périmètre de la deuxième partie (2).

8. Timbre de préparation de la peau selon la revendication 6 ou la revendication 7, dans lequel au moins les première et deuxième parties (1, 2) du timbre comprennent la couche adhésive.

9. Timbre de préparation de la peau selon l'une quelconque des revendications 6 à 8, dans lequel la première partie (1) du timbre entoure la deuxième partie (2).

10. Timbre de préparation de la peau selon l'une quelconque des revendications 6 à 9, dans lequel la première partie (1) du timbre comprend au moins un coin interne pour définir l'emplacement souhaité du dispositif transdermique (10) à placer en contact avec la peau (8).
